# EUROPEAN PATENT SPECIFICATION

(11) **EP 3 650 082 B1**
(45) Date of publication and mention of the grant of the patent: **20.03.2024**
(21) Application number: 18204868.6
(22) Date of filing: 07.11.2018
(51) Int. Cl.: A61Q 1/00, A61Q 19/00, A61K 8/02, A61Q 1/14, A61K 8/34, A61K 8/35, A61K 8/49, A61K 8/81, A61K 8/92

(54) **DISPERSION INCLUDING GLITTERS FOR THE PREPARATION OF HUMIDIFYING WIPES**
DISPERSION MIT GLITTER ZUR HERSTELLUNG VON BEFEUCHTUNGSTÜCHERN
DISPERSION CONTENANT DES PAILLETTES POUR LA PRÉPARATION DE LINGETTES HUMIDIFIANTES

(43) Date of publication of application: 13.05.2020
(73) Proprietor: O-pac S.r.l. Società A Socio Unico, 23848 Oggiono, Lecco (IT)
(72) Inventor: BARTESAGHI, Antonio, I-23848 OGGIONO, LECCO (IT); CAVENAGHI, Roberto, I-23848 OGGIONO, LECCO (IT)
(74) Representative: Croce, Valeria

(56) References cited:
- WO-A2-2007/069214
- US-A1- 2005 002 994
- US-B2- 8 435 625
- DATABASE GNPD [Online] MINTEL; 25 October 2016 (2016-10-25), anonymous: "Tear Drop Gel Cream", XP55588567, retrieved from www.gnpd.com Database accession no. 4356343
- DATABASE GNPD [Online] MINTEL; 8 May 2010 (2010-05-08), anonymous: "Topical Gel", XP55588618, retrieved from www.gnpd.com Database accession no. 1294805

## Description

The present invention relates to the field of cosmetics, in particular for preparing the so-called wet wipes.

It is a highly appreciated consumer good.

Several types exist on the market, which range from personal hygiene wipes for children, to those for cleaning hands, up to make-up removing wipes or wipes soaked in insect repelling substances.

The product is available in individual packages or often in packs, from which the wipes may be extracted individually.

In any case, the production process should always ensure obtaining wipes characterized by a standard quality.

Use was made to date of processes which were capable of producing wipes having a homogenous aspect and qualities, in some manner excluding certain possible applications.

Prior-art document US 2005/002994 discloses water-insoluble nonwoven materials impregnated with highly liquid cosmetic and dermatological impregnation solutions in particular with highly liquid cosmetic and dermatological water-in-oil emulsions (W/O emulsions) which are long-term stable. Said solutions may comprise effect pigments or glitter as mixtures with various excipients and dyes.

### Summary of the invention

The authors of the present invention have surprisingly found that a composition for preparing wet wipes comprising a particulate matter may be achieved.

### Subject of the invention

The present invention relates to a composition for preparing wet wipes comprising particulate matter according to claim 1 and dependent claims thereof.

The method for obtaining wet wipes represents a further subject of the present invention according to claim 5 and dependent claims thereof.

### Detailed description of the invention

The percentage compositions in the present description are indicated by weight with respect to the total weight of the composition.

According to a first object of the invention, a composition for preparing wet wipes is described.

In a first aspect, the composition is a cosmetic composition, possibly with decorative or make-up function, but it is not excluded for the compositions to be prepared for other applications.

According to a first aspect, the composition of the invention comprises a moistening agent, a lipophilic solubilizing agent, an emulsifying agent, a preserving agent, a viscosifying and/or stabilizing agent.

The composition further comprises a pH modifying agent.

As far as the solvent is concerned, this is an hydroalcoholic solvent.

The alcohol is ethyl alcohol or denatured ethyl alcohol (all types of denaturing allowed).

For the purposes of the present invention, the moistening agent is present in amounts from about 0.1 to 1% (w/w).

The moistening agent is methylpropanediol. For the purposes of the present invention, a lipophilic solubilizing agent is present in amounts from about 0.1 to 1 (w/w).

The lipophilic solubilizing agent is PEG-40 hydrogenated castor oil.

For the purposes of the present invention, an emulsifying agent is present in amounts from about 0.1 to 1 (w/w).

The emulsifying agent is polysorbate 20.

For the purposes of the present invention, the preserving agent is present in amounts from about 0.2 to 2 (w/w).

The preserving agent is a mixture of phenoxyethanol and ethylhexylglycerin

For example, each of the phenoxyethanol and ethylhexylglycerin may be present in an amount from about 0.1 to 1% (w/w).

For the purposes of the present invention, the viscosifying agent is present in amounts from about 0.1 to 1 (w/w).

The viscosifying agent is an acrylate copolymer/palmeth-25 acrylate (CAS 330991-05-4), and in a preferred aspect, Synthalen^{®} W2000 (3V Group).

More generally, pseudoplastic rheological modifiers may be used for the same purpose.

The pH modifying agent preferably is a sodium hydroxide solution; for example, a 40% (w/w) soda solution may be used.

The pH modifying agent is added in sufficient amounts so that the pH of the end composition is from about 7.2 to 7.8; an amount of about ≤0.10 % (w/w) is normally used.

The composition of the invention may also comprise a fragrance or scent, selected by those skilled in the art according to commercial needs.

The composition described has a density from about 1.0 to 1.05 for the purposes of the present invention.

The term "particulate" means a particulate matter of various shape which is insoluble in the composition of the invention.

Particularly, such a particulate has a size from about 0.010 to 5 mm and preferably from about 0.02 to 0.2 mm.

In particular, the particular size is measured by laser diffraction (as per the supplier specifications), or alternatively, by sieving.

Preferably, the particulate dispersed in the composition of the invention is pearls, beads, glitter, scrub.

For the purposes of the present invention, the particulate may be of natural or synthetic origin, possibly biocompatible and/or recyclable.

In particular, the particulate is a pigment.

The particulate may be present in an amount up to about 10% (w/w).

According to one embodiment of the present invention, the pigment comprises calcium aluminum borosilicate, titanium dioxide, silica and titanium oxide.

In a preferred aspect, a pigment may be used, comprising:

| **Component** | **Amount (w/w)** |
|---|---|
| Calcium aluminum borosilicate | 0.1-1 |
| Silica | ≤0.10 |
| Titanium dioxide | ≤0.10 |
| Titanium dioxide | ≤0.10 |

In a more preferred aspect of the invention, the composition of the invention comprises:

| **Component** | **Concentration (w/w)** |
|---|---|
| Water | 75-100 |
| Ethanol | 5-10 |
| Methylpropanediol | 0.1-1 |
| PEG-40 hydrogenated castor oil | 0.1-1 |
| Polysorbate 20 | 0.1-1 |
| Phenoxyethanol | 0.1-1 |
| Ethylhexylglycerin | 0.1-1 |
| Particulate | ≤10 |
| Acrylate copolymer/palmeth-25 acrylate | 0.1-1 |
| pH modifying agent | ≤0.10 |

A process for preparing the above-mentioned cosmetic composition is below reported.

As described above, such a composition is intended to be applied to wet wipes.

For the purposes of the present invention, within the present description, the process comprises the steps of:
A) preparing the solvent,
B) preparing a mixture of the moistening agent, the lipophilic solubilizing agent, the emulsifier, the preserving agent and lastly, the particulate matter,
C) adding the mixture of step B) to the solvent of step A),
D) adding the viscosifying agent and the p_{H} modifying agent.

As described above, in a preferred aspect, the solvent is a hydroalcoholic solvent comprising water:ethanol in a ratio from about 7:1 to 9:1.

The mixture of step B) comprises the above-indicated components and is prepared by mixing so as to obtain a homogenous suspension.

In step D), the viscosifying agent and the pH modifying agent are added according as indicated above.

The present invention also describes a method for preparing wet wipes comprising the composition detailed above.

In particular, such a method comprises the steps of:
1) preparing a cosmetic composition according to the invention, and
2) applying such a composition to a support according to the invention by means of soaking.

For the purposes of the present invention, the term "support" in step 2 means a fabric or non-woven sheet, made of natural or synthetic material, to which the composition of the present Patent Application is applied.

According to an aspect of the invention, the support is made of viscose, cotton, polyester, Lyocell^{®}, Bioflush, cellulose, polypropylene, bamboo.

In particular, the support may be made of a pure material or a more or less varied mixture of various materials.

The support material is white, but it may possibly be colored.

In a preferred aspect of the invention, step 2) is performed only on the upper face of the support.

The support thus soaked with the composition of the invention is then subjected to the steps of cutting and folding.

Obviously, if the soaking is performed on the individual supports, the next cutting step is not required.

In an alternative aspect of the invention, the support may first be folded and then subjected to cutting.

In any case, the step of folding the support is preferably performed in a symmetrical manner so that there are no portions of the upper surface of the support remaining exposed.

The supports thus obtained may then be conveniently packaged, individually (for example, in disposable packages) or in multiples (for example, in packs).

In a preferred aspect of the invention, if the supports in the shape of wet wipes are packaged in multiples, the folded supports, possibly overlapping one another, are not interleaved with one another.

Thereby, the user may advantageously extract the wipe from the container without the particulate being applied to the user's hands.

The present invention is described in detail below by means of application examples.

### Example 1

A composition according to the present invention comprises:

| **Component** | | **Concentration % (w/w)** |
|---|---|---|
| Water | | 85.68 |
| Ethanol | | 10 |
| Methylpropanediol | Dub Diol | 1 |
| PEG-40 hydrogenated castor oil | Cremophor RH40 | 0.3 |
| Polysorbate 20 | Polysorbate 20 | 0.3 |
| Fragrance | Lindenapple FH10693/4 | 0.2 |
| Phenoxyethanol Ethylhexylglycerin | Euxyl PE 9010 | 1.0 |
| Calcium aluminum borosilicate, titanium dioxide, silica, titanium oxide | Ronastar^{®} Copper Sparks | 0.3 |
| Acrylate copolymer/palmeth-25 acrylate | Synthalen W2000 | 1.1 |
| Water, sodium hydroxide | Soda 40% | 0.120 |

The composition has the following features:

| Aspect | Solution with suspended pearl |
|---|---|
| Color | Pearlescent |
| Smell | Characteristic |
| pH | 7.20-7.80 |
| Density | 1.0-1.05 |

The many advantages provided by the present invention will become apparent to those skilled in the art from the reading of the aforementioned description.

The fine-tuned composition allows to keep the particulate matter - represented by beads or glitter
- distributed in a homogeneous manner on the wet wipe.

The process for preparing the wipes also allows the particulate matter to be deposited uniformly on the surface thereof.

In the embodiment in which the wipes are folded in a symmetrical manner, the user is advantageously able to avoid soiling his/her hands with the particulate matter.

Lastly, the described solution allows the range of cosmetic products which may be offered on the market to be greatly expanded.

## Claims

1. A cosmetic composition to be applied to wet wipes comprising
| **Component** | **Concentration (w/w)** |
|---|---|
| Water | 75-100 |
| Ethanol | 5-10 |
| Methylpropanediol | 0.1-1 |
| PEG-40 hydrogenated castor oil | 0.1-1 |
| Polysorbate 20 | 0.1-1 |
| Phenoxyethanol | 0.1-1 |
| Ethylhexylglycerin | 0.1-1 |
| Particulate | ≤10 |
| Acrylate copolymer/palmeth-25 acrylate | 0.1-1 |
| pH modifying agent | ≤0.10 |
**characterized in that** said pH modifying agent is added in sufficient amounts so that the pH of the end composition is from about 7.2 to 7.8 and **in that** said particulate has a size from about 0.01 to 5 mm, wherein the composition comprises the particulate and the pH modifying agent.

2. A cosmetic composition according to the preceding claim, wherein the particulate matter is a pigment, glitter, beads.

3. A cosmetic composition according to claim 1 or 2, wherein said particulate has a size from about 0.02 to 0.2 mm.

4. A cosmetic composition according to any one of claims 1 to 3, having a density from about 1.0 to 1.05.

5. A method for preparing wet wipes comprising the steps of:
a) preparing a cosmetic composition according to any one of the preceding claims 1 to 4,
b) applying such a composition to a support by means of soaking.

6. A method according to the preceding claim, wherein step b) is only performed on the upper face of said support.

7. A method according to claim 5 or 6, further comprising the step c) of folding such a support.

8. A method according to any one of claims 5 to 7, wherein after such a step c), the support material is symmetrically folded so that the soaked upper face is completely folded inwards.

9. A method according to any one of claims 5 to 8, wherein the support of step b) is a fabric or a non-woven fabric made of natural and/or synthetic material.

10. A method according to the preceding claim, wherein said support is made of viscose, cotton, polyester, cellulose, polypropylene, bamboo, either intended as a pure material or as a mixture of various materials.

## Patentansprüche

1. Kosmetische Zusammensetzung zum Aufbringen auf Feuchttücher, umfassend
| **Bestandteil** | **Konzentration (Gew./Gew.)** |
|---|---|
| Wasser | 75-100 |
| Ethanol | 5-10 |
| Methylpropandiol | 0,1-1 |
| PEG-40 hydriertes Rizinusö | 0,1-1 |
| Polysorbat 20 | 0,1-1 |
| Phenoxyethanol | 0,1-1 |
| Ethylhexylglycerin | 0,1-1 |
| Partikel | ≤ 10 |
| Acrylat-Copolymer/Palmeth-25-Acrylat | 0,1-1 |
| pH-Modifizierungsmittel | ≤ 0,10 |
**dadurch gekennzeichnet, dass** das pH-Modifizierungsmittel in ausreichenden Mengen zugesetzt wird, so dass der pH-Wert der Endzusammensetzung etwa 7,2 bis 7,8 beträgt, und dass die Partikel eine Größe von etwa 0,01 bis 5 mm haben, wobei die Zusammensetzung die Partikel und das pH-Modifizierungsmittel umfasst.

2. Kosmetische Zusammensetzung nach dem vorhergehenden Anspruch, wobei die partikelförmige Substanz ein Pigment, Glitter, Perlen ist.

3. Kosmetische Zusammensetzung nach Anspruch 1 oder 2, wobei die Partikel eine Größe von etwa 0,02 bis 0,2 mm haben.

4. Kosmetische Zusammensetzung nach einem der Ansprüche 1 bis 3, die eine Dichte von etwa 1,0 bis 1,05 aufweist.

5. Verfahren zur Herstellung von Feuchttüchern, umfassend die Schritte:
a) Herstellen einer kosmetischen Zusammensetzung nach einem der vorhergehenden Ansprüche 1 bis 4,
b) Aufbringen einer solchen Zusammensetzung auf einen Träger durch Tränken.

6. Verfahren nach dem vorhergehenden Anspruch, wobei Schritt b) nur auf der Oberseite des Trägers durchgeführt wird.

7. Verfahren nach Anspruch 5 oder 6, ferner umfassend den Schritt c) des Faltens eines solchen Trägers.

8. Verfahren nach einem der Ansprüche 5 bis 7, wobei nach einem solchen Schritt c) das Trägermaterial symmetrisch gefaltet wird, so dass die getränkte Oberseite vollständig nach innen gefaltet wird.

9. Verfahren nach einem der Ansprüche 5 bis 8, wobei der Träger von Schritt b) ein Gewebe oder ein Vlies aus natürlichem und/oder synthetischem Material ist.

10. Verfahren nach dem vorhergehenden Anspruch, wobei der Träger aus Viskose, Baumwolle, Polyester, Zellulose, Polypropylen, Bambus, entweder als reines Material oder als Mischung verschiedener Materialien, hergestellt ist.

## Revendications

1. Composition cosmétique à appliquer à des lingettes humides comprenant
| **Composant** | **Concentration (p/p)** |
|---|---|
| Eau | 75-100 |
| Éthanol | 5-10 |
| Méthylpropanediol | 0,1-1 |
| Huile de ricin hydrogénée PEG-40 | 0,1-1 |
| Polysorbate 20 | 0,1-1 |
| Phénoxyéthanol | 0,1-1 |
| Éthylhexylglycérine | 0,1-1 |
| Particule | ≤ 10 |
| Copolymère d'acrylate/acrylate palmeth-25 | 0,1-1 |
| Agent de modification du pH | ≤ 0,10 |
**caractérisée en ce que** ledit agent de modification du pH est ajouté en quantités suffisantes de telle sorte que le pH de la composition finale soit d'environ 7,2 à 7,8 et **en ce que** ladite particule présente une taille d'environ 0,01 à 5 mm, dans laquelle la composition comprend la particule et l'agent de modification du pH.

2. Composition cosmétique selon la revendication précédente, dans laquelle la matière particulaire est un pigment, des paillettes, des perles.

3. Composition cosmétique selon la revendication 1 ou 2, dans laquelle ladite particule présente une taille d'environ 0,02 à 0,2 mm.

4. Composition cosmétique selon l'une quelconque des revendications 1 à 3, présentant une densité d'environ 1,0 à 1,05.

5. Procédé de préparation de lingettes humides comprenant les étapes :
a) de préparation d'une composition cosmétique selon l'une quelconque des revendications précédentes 1 à 4,
b) d'application d'une telle composition à un support par trempage.

6. Procédé selon la revendication précédente, dans lequel l'étape b) est réalisée seulement sur la face supérieure dudit support.

7. Procédé selon la revendication 5 ou 6, comprenant en outre l'étape c) de pliage d'un tel support.

8. Procédé selon l'une quelconque des revendications 5 à 7, dans lequel après une telle étape c), le matériau de support est plié de manière symétrique de telle sorte que la face supérieure trempée est complètement pliée vers l'intérieur.

9. Procédé selon l'une quelconque des revendications 5 à 8, dans lequel le support de l'étape b) est un tissu ou un tissu non tissé composé de matière naturelle et/ou synthétique.

10. Procédé selon la revendication précédente, dans lequel ledit support est composé de viscose, de coton, de polyester, de cellulose, de polypropylène, de bambou, prévu soit en tant que matière pure soit en tant que mélange de diverses matières.
